Europäisches Patentamt

European Patent Office

Office européen des brevets

(19)

(11) Veröffentlichungsnummer: **0 267 978 B1**

(12) **EUROPÄISCHE PATENTSCHRIFT**

(45) Veröffentlichungstag der Patentschrift: 28.08.91

(51) Int. Cl.5: **A61B 5/00**

(21) Anmeldenummer: 86115941.6

(22) Anmeldetag: 17.11.86

(54) **Kombinationssensor zur transcutanen Erfassung von Sauerstoff und Kohlendioxid im Blut.**

(43) Veröffentlichungstag der Anmeldung:
25.05.88 Patentblatt 88/21

(45) Bekanntmachung des Hinweises auf die
Patenterteilung:
28.08.91 Patentblatt 91/35

(84) Benannte Vertragsstaaten:
CH DE FR GB LI

(56) Entgegenhaltungen:
EP-A- 0 104 772       DE-A- 3 232 515
GB-A- 2 033 575       GB-A- 2 054 844
US-A- 3 769 974       US-A- 4 557 900

IEEE TRANSACTIONS ON BIOMEDICAL ENGI-
NEERING, Band BME-31, Nr. 12, Dezember
1984, Seiten 792-800, IEEE, New York, US; Y.
MENDELSON et al.: "Noninvasive transcutaneous monitoring of arterial blood gases"

(73) Patentinhaber: PPG Hellige GmbH
Heinrich-von-Stephan-Str. 4
W-7800 Freiburg(DE)

(72) Erfinder: Ullrich, Georg J., Dipl.-Phys.
Mettackerweg 84
W-7800 Freiburg i.Br.(DE)

(74) Vertreter: TER MEER - MÜLLER - STEINMEI-
STER & PARTNER
Mauerkircherstrasse 45
W-8000 München 80(DE)

EP 0 267 978 B1

## Beschreibung

Die Erfindung betrifft einen Kombinationssensor zur transcutanen Erfassung von Sauerstoff (O₂) und Kohlendioxid (CO₂) im Blut.

Meßaufnehmer zur transcutanen Überwachung der Blutgase O₂ und/oder CO₂ sind bekannt. Für solche auch als Meßfühler oder kurz Sensoren bezeichnete Meßaufnehmer ist es aus der DE-C3-21 45 400 auch bekannt, einen als Referenz- oder Gegenelektrode dienenden Metallkörper elektrisch zu beheizen und über eine Regeleinrichtung auf einer konstanten Temperatur zu halten. Die elektrische Beheizung hat hierbei zwei Funktionen. Zum einen erzeugt die Erwärmung eine Hyperämie von Haut und Gewebe unter dem Meßfühler und zum anderen bewirkt die Konstanthaltung der Temperatur des Meßfühlers eine Stabilisierung der Eigenschaften der in ihm enthaltenen physikalischen oder elektrochemischen Meßelemente. Außerdem kann unter gewissen Bedingungen die für die Aufrechterhaltung der Temperatur des Meßfühlers erforderliche Heizleistung zur Überwachung der relativen lokalen Durchblutung herangezogen werden; vgl. Buch HUCH/HUCH/LÜBBERS: Transcutaneous pO₂; THIEME VERLAG Stuttgart-New York 1981. Im gleichen Buch sind auf Seite 101 bis 107 Sensoren und Meßeinrichtungen zur transcutanen Überwachung des Sauerstoffpartialdrucks pO₂ beschrieben, bei denen der Sauerstoff polarographisch gemessen wird, und auf Seite 78 bis 80 wird darauf hingewiesen, daß eine ausreichende Sensortemperatur für die Validität der transcutanen pO₂-Überwachung erforderlich ist. Als Mindesttemperatur für diesen Zweck gilt 43° C; angewandt werden üblicherweise Temperaturen bis 45° C.

Um Hautschäden bei diesen Temperaturen zu vermeiden, muß der Meßaufnehmer von Zeit zu Zeit auf eine andere Stelle der Hautoberfläche gesetzt werden; bei einer Betriebstemperatur von 43° C etwa alle 4 Stunden. Dieser Umstand erfordert Aufmerksamkeit und Betreuung durch verläßliches Personal wie es im häuslichen Bereich erfahrungsgemäß nicht vorausgesetzt werden kann.

Um eine solche Überwachung dennoch durchführen zu können, wird ein Verfahren praktiziert, bei dem mit zwei Aufnehmern gearbeitet wird, die abwechselnd für eine gewisse Zeit beheizt und zur pO₂-Messung benutzt werden; vgl. Aufsatz PETER, H.J.: Holter Monitoring Technique in a Comprehensive Approach: Ambulatory Monitoring of Sleep Apnea; in Holter Monitoring Technique, Hergb.: HOMBACH/HILGER; VERLAG SCHATTAUER Stuttgart-New York 1985, Seite 134/135. Diese Lösung hat jedoch die Nachteile, daß zwei Sensoren benötigt werden, was insbesondere bei kleinen Kindern zu Schwierigkeiten führt, und daß komplizierte elektronische Mittel erforderlich sind, die in den vorbestimmten Zeitabständen zwischen den Meßfühlern umschalten. Außerdem ergeben sich Probleme aus der erforderlichen gleichen Eichung der beiden Sensoren.

Meßfühler zur transcutanen Überwachung des Kohlendioxids (CO₂) sind beispielsweise in der DE-A1-29 11 343 sowie in der DE-A1-32 32 515 beschrieben. Diese Meßaufnehmer enthalten pH-Meßelektroden, die den pH-Wert in einer dünnen Schicht einer Elektrolytlösung messen, die durch eine CO₂-durchlässige Membran abgeschlossen ist und über CO₂-Gasdiffusion im Gasaustausch mit dem Meßgut steht. Der pH-Wert wird hierbei entweder, wie bei Blutgasanalysatoren seit langem bekannt, mit der bekannten Glaselektrode gemessen, z. B. in der Ausführungsform gemäß DE-A1-29 11 343 oder neuerdings mit einer speziellen Iridium-Iridiumoxid-Elektrode, wie in der DE-A1-32 32 515 beschrieben.

Wünschenswert und in kritischen Fällen erforderlich ist es sowohl den Sauerstoff als auch das Kohlendioxid zu überwachen. Demgemäß ist aus der DE-A1-23 05 049 der an sich naheliegende Vorschlag bekannt, die Sensoren für pO₂ und pCO₂ räumlich in einem Sensorgehäuse zu kombinieren. Dieser Vorschlag hat inzwischen zu pO₂-pCO₂-Kombinationssensoren geführt, die, um eine befriedigende pO₂-Messung sicherzustellen, ebenfalls auf mindestens 43° C beheizt werden müssen. Außerdem ist für den pCO₂-Meßteil, der beispielsweise gemäß DE-A1-23 05 049 durch eine Glaselektrode verwirklicht ist, eine Nachkalibrierung in bestimmten Zeitabständen erforderlich.

Der Erfindung liegt die Aufgabe zugrunde, einen Kombinationssensor bzw. kombinierten Meßfühler zur gleichzeitigen fortlaufenden transcutanen Messung von Kohlendioxid und Sauerstoff im Blut für längere Zeit, z. B. über einen Tag oder eine Nacht hinweg, mit einem einzigen Sensor zu überwachen, ohne daß diese Überwachung zum Umsetzen des Sensors, zur Umschaltung auf einen anderen Sensor oder für Kalibriermaßnahmen unterbrochen werden muß.

Ein Bedarf für eine Lösung dieser Aufgabe besteht z. B. bei der Überwachung von Patienten mit nächtlichen Atem-Störungen oder von Kindern, die vom sogenannten plötzlichen Kindstod (Sudden Infant Death Syndrom = SIDS) bedroht sind. Es hat sich nämlich gezeigt, daß solche Patienten möglichst auch in ihrer natürlichen Umgebung im häuslichen Bereich überwacht werden sollten, sei es zur genaueren diagnostischen Abklärung ihres Leidens oder in einer Phase des Übergangs von der klinischen Betreuung in das häusliche Milieu, in der es aus Sicherheitsgründen angezeigt erscheint, die in der Klinik durchgeführte Überwachung in einem gewissen Mindestumfang zu Hause fortzusetzen. Überwachung dieser Art im häuslichen Bereich erfordert aber eine Meßeinrichtung, mit der auch der Laie zurechtkommt, ohne daß eine unzuverlässige Messung oder eine Gefährdung des Patienten befürchtet werden muß.

Entsprechend der Lehre des Patentanspruchs 1 basiert die erfindungsgemäße Lösung auf der Idee, die Möglichkeit einer ununterbrochenen transcutanen $pCO_2$-Überwachung über längere Zeit für einen kombinierten transcutan messenden Sauerstoff/KohlendioxidSensor dadurch nutzbar zu machen, daß - abweichend von bisher bekannten Lösungen - in einem Meßaufnehmer eine $pCO_2$-Meßeinrichtung nicht mit einer solchen zur Messung des Sauerstoffpartialdrucks $pO_2$, sondern mit einer Meßeinrichtung zur spektrophotometrischen Messung der Sauerstoffsättigung des Bluts $sO_2$ kombiniert wird. Damit ist zunächst das Problem der lokalen Überhitzung der Meßstelle auf der Haut beseitigt, da eine Erwärmung der Haut auf 43°C oder mehr nicht mehr erforderlich ist.

Das Problem der bisher erforderlichen Zwischenkalibrierung des Sensorteils für die $pCO_2$-Überwachung konnte durch die überraschende Feststellung gelöst werden, daß Meßaufnehmer zur transcutanen $pCO_2$-Messung, wie sie in der DE-A1-32 32 515 beschrieben sind, die also mit einer Iridium-Iridiumoxid-Elektrode für die pH-Wertmessung ausgerüstet sind, so driftarm sind, daß sie ohne Zwischenkalibrierung, d. h. ohne Unterbrechung der Überwachung, über 24 Stunden hinweg bei niedrigerer Betriebstemperatur von beispielsweise 42°C betrieben und auf einer Hautstelle des Patienten ohne Hautschäden belassen werden können.

Weiterhin hat sich gezeigt, daß in vielen Fällen die Messung der Sauerstoffsättigung des Bluts $sO_2$ zur Überwachung von Patienten ausreicht. Dies gilt insbesondere für das beschriebene Anwendungsgebiet im häuslichen Bereich z. B. bei der Überwachung SIDS-gefährdeter Kinder oder durch nächtliche Apnoe gefährdeter Patienten.

Entsprechend einer Ausführungsform der Erfindung enthält der Kombinationssensor zur transcutanen Erfassung von Sauerstoff ($O_2$) und Kohlendioxid ($CO_2$) im Blut eine Meßvorrichtung für $pCO_2$ mit einer Iridium/Iridiumoxid-Meßelektrode und eine Meßvorrichtung zur spektrophotometrischen Bestimmung der Sauerstoffsättigung $sO_2$, die aus einer Kombination von lichtemittierenden Dioden, die Licht unterschiedlicher Wellenlängen abstrahlen und einem oder mehreren zugehörigen photoelektrischen Empfängern bestehen kann. Die Zuführung und Abführung des Lichts kann auch über Lichtleiter erfolgen, wobei die spektrale Lichterzeugung und -messung im Auswertegerät erfolgt. Die Wellenlängen der beiden lichtemittierenden Dioden werden bei der bevorzugten Ausführungsform der Erfindung einmal im Infrarotbereich, insbesondere isosbestisch für Hämoglobin bzw. Oxyhämoglobin und vorzugsweise zu $\lambda$ = 805 nm und die andere Wellenlänge im Rotbereich insbesondere zu etwa $\lambda$ = 650 nm gewählt.

Besonders zweckmäßig ist es, der Signalauswertung das Prinzip der Pulsoxymetrie zugrunde zu legen, d. h. die lichtemittierenden Dioden und/oder die Signalauswertung intermittierend und angepaßt auf die Pulsphasen der arteriellen Füllung des Meßareals vorzunehmen. Dieses Prinzip ist an sich bekannt und beispielsweise in einem Aufsatz Yoshia/Shimada/Tanaka: Spectrophotometric Monitoring of Arterial Oxygen Saturation in the Fingertip, Med. Biol. Engng. Comput. 18:27-32 (1980) beschrieben. Eine frühere grundsätzliche Darstellung des Verfahrens der Oxymetrie und seiner historischen Entwicklung findet sich z. B. bei ULLRICH: Physikalisch-technisches zur Oxymetrie und Farbstoffinjektionsmethode; HELLIGE-Mitteilungen für die Medizin, Nr. 7, Seite 4 bis 16 (1964).

Mit der Erfindung wurde also ein kombinierter Meßfühler oder Sensor zur gleichzeitigen fortlaufenden transcutanen Messung des Partialdrucks von Kohlendioxid im Blut ($tcpCO_2$) sowie der Sauerstoffsättigung ($tcsO_2$) des Bluts geschaffen, der wie bekannte kleine Sensoren auf die Haut geklebt werden kann und lediglich auf eine Temperatur von 42°C oder weniger erwärmt und thermostatisiert zu werden braucht, wobei bei diesen Betriebstemperaturen der zusätzliche Vorteil erreicht wird, daß die Qualität des pulsoxymetrischen Meßsignals deutlich verbessert und stabilisiert wird.

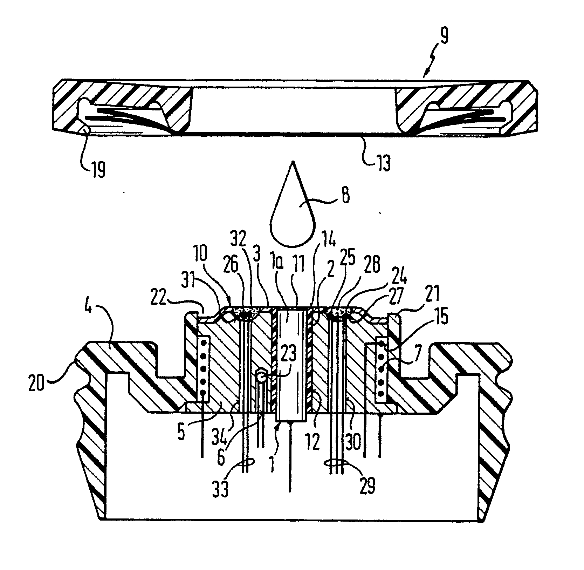

Die Erfindung und vorteilhafte Einzelheiten werden nachfolgend anhand eines Ausführungsbeispiels unter Bezug auf die Zeichnung näher erläutert. Die einzige Figur zeigt in schematischer Darstellung das Schnittbild eines erfindungsgemäßen Kombinationssensors.

Die Zeichnung läßt ein ringförmiges Gehäuse 4 aus Kunststoff erkennen mit einem mittigen Durchbruch 15, in den ein Metallkörper 5 vorzugsweise aus Silber (Ag) paßgenau eingesetzt ist, der in der Darstellung oberseitig, d. h. die (spätere) Meßfläche 10 überdeckend mit einer SilberSilberchlorid-Referenzelektrode 3 versehen ist. Der Durchbruch 15 wird durch eine zylindrische Wandung 21 begrenzt, die umlaufend geringfügig über die Oberfläche der Referenzelektrode 3 übersteht, so daß zusätzlich, bedingt durch eine randseitige Zurücksetzung der Referenzelektrode 3, ein Elektrolytreservoir 22 gebildet ist. Der Metallkörper 5 weist, die Referenzelektrode 3 ebenfalls durchsetzend, eine zentrale Bohrung 12 auf, in die in konzentrischer Anordnung eine pH-Meßelektrode 1 eingepaßt ist, welche bei der bevorzugten Ausführungsform eine Iridium/Iridiumoxid(Ir/IrOx-)-Elektrode ist, die beispielsweise einen Durchmesser von 2 mm aufweist. Diese Elektrode 1 ist gegen die Referenzelektrode 3 sowie gegen den Metallkörper 5 durch eine Kunststoff-Gießharzschicht 2 isoliert. Wie aus der DE-A1-32 32 515 bekannt, kann die Ir/IrOx-Elektrode 1 aus einem einheitlichen zylinderförmigen Iridiumstück hergestellt sein, das oberflächenseitig, also auf der einer Membran 13 zugekehrten und in einen Elektrolyten 8 eintauchenden Oberfläche oxidiert ist. Iridium ist jedoch ein vergleichsweise sehr teures Metall. Aus diesem Grund wird es schon aus Preisgründen in der Regel vorteilhaft sein, den Körper der Ir/IrOx-Elektrode 1 stirnseitig, d. h. auf der in den Elektrolyten 8 eintauchenden Oberfläche, mit einem Iridiumplättchen 11 zu versehen, das mit dem übrigen Elektrodenkörper la, der beispielsweise aus Silber oder Kupfer bestehen kann, elektrisch und thermisch gut leitend verbunden ist. Die in den Elektrolyten 8 eintauchende Oberfläche des Iridiumplättchens 11 ist in diesem Fall elektrochemisch oder thermochemisch oxidiert, wobei das Oxid auch in hydratisierter Form als Iridiumoxidhydrat vorliegen kann. Die Referenzelektrode 3 ist entweder durch eine zumindest teilweise chlorierte Oberfläche des aus Silber bestehenden Metallkörpers 5 gebildet oder als ringförmiges Plättchen aus Silber/Silberchlorid-Sintermetall auf den Metallkörper 5 aufplattiert. Der Metallkörper 5 ist im Bereich seiner umlaufenden Mantelfläche mit einer ringförmig umlaufenden breiten Nut versehen, in die eine Heizwicklung 7 eingesetzt ist. Die Heizleistungszufuhr zum Metallkörper 5 wird über wenigstens einen in eine exzentrische Bohrung 6 eingesetzten Temperaturfühler, beispielsweise einen Thermistor 23, geregelt. In an sich bekannter Weise kann ein weiterer (nicht gezeigter) Temperaturfühler, wiederum beispielsweise ein Thermistor, in den Metallkörper 5 eingesetzt sein, der ein (nicht gezeigtes) Schalterelement zur unmittelbaren Unterbrechung der Heizleistungszufuhr zur Heizwicklung 7 steuert, wenn die Temperatur im Metallkörper einen vorgebbaren Schwellenwert von beispielsweise 42°C überschreitet.

Die so weit beschriebene und an sich beispielsweise aus der DE-A1-32 32 515 bekannte pCO$_2$-Meßvorrichtung mit pH-Meßelektrode ist erfindungsgemäß mit einer sO$_2$-Meßvorrichtungkombiniert, die bei der dargestellten Ausführungsform einerseits aus zwei photoelektrischen Senderelementen in Form zweier lichtemittierender Dioden 24 und 25 und andererseits aus einem photoelektrischen Empfängerelement 26 besteht, das vorzugsweise ein Silicium-Photoelement (Si-Photoelement) sein wird. Die beiden lichtemittierenden Diodenelemente 24 und 25 sind in eine muldenartige Aussparung 27 in der Schicht aus Ag/AgCl der Referenzelektrode 3 bzw. des Metallkörpers 5 eingesetzt, die mit einer lichtdurchlässigen Vergußmasse 28 feuchtigkeits- und luftdicht ausgegossen ist, die oberflächenseitig, d.h. auf der der Membran 13 zugekehrten Meßflächenseite, plan verschliffen und poliert ist. Die lichtdurchlässige Vergußmasse 28 kann gleichzeitig als (zusätzliche) Passivierungsschicht für die lichtemittierenden Dioden 24 und 25 dienen. Die Lichtaustrittsseiten der Dioden 24 und 25 sind auf die nach der weiter unten beschriebenen Vervollständigung des Sensors zwischen der Meßfläche und der Membran vorhandenen Schicht des Elektrolyten 8 so ausgerichtet, daß sich ihre Strahlen nicht gegenseitig stören. Die Signalzuführung zu den Dioden 24 und 25 erfolgt über Zuleitungsdrähte 29, welche eine Bohrung 30 im Metallkörper 5 durchsetzen. Die Bohrung 30 kann ebenfalls mit einem geeigneten Gießharz ausgefüllt sein.

Das photoelektrische Empfängerelement 26 ist in ähnlicher Weise wie die Dioden 24 und 25 in eine muldenartige Aussparung 31 mittels einer ebenfalls lichtdurchlässigen Vergußmasse 32 in Ausrichtung auf die Meßfläche bzw. die Membran 13 und die (spätere) Elektrolytschicht 8 eingesetzt. Die Signalabnahme erfolgt über Leitungsdrähte 33, welche eine weitere, im Endzustand des Sensors ebenfalls mit ausgehärtetem Gießharz gefüllte Bohrung 34 im Metallkörper 5 durchsetzen.

Die beiden lichtemittierenden Dioden 24 und 25 geben bei entsprechender Erregung Licht unterschiedlicher Wellenlänge ab. Eine dieser Wellenlängen wird in Anpassung auf den Extinktionskoeffizienten von Hämoglobin bzw. Oxyhämoglobin oder in anderen Worten isosbestisch für Hämoglobin bzw. Oxyhämoglobin im Ultrarotbereich und insbesondere zu $\lambda$ = 805 nm gewählt, während die Wellenlänge für die andere Diode im Rotbereich beispielsweise bei etwa $\lambda$ = 650 nm gewählt wird. Die Ansteuerung der beiden lichtemittierenden Dioden 24 und 25 bzw. die Signalauswertung erfolgt zweckmäßigerweise, wie bereits oben erwähnt, nach dem Prinzip der Pulsoxymetrie.

Um den Sensor für einen Meßvorgang vorzubereiten wird, wie die Zeichnung erkennen läßt, ein kleiner Tropfen des Elektrolyten 8 auf die durchgehende Oberfläche 14 (Meßfläche) der Referenzelektrode 3, der oberflächenseitig polierten lichtdurchlässigen Vergußmassen 28 und 32 sowie der Ir/IrOx-Elektrode 1 aufgebracht. Sodann wird der Kombinationssensor mit einer vorgefertigten einmal zu benutzenden Schnappring-Membranvorrichtung 9 bespannt. Der leicht gewölbt gefertigte Schnappring 9 weist eine nach innen vorspringende umlaufende Rastkante 19 auf. Wird der Schnappring 9 auf das Gehäuse 4 aufgedrückt, so rastet die Rastkante 19 hinter einem vorspringenden Rand 20 am Gehäuse ein und die für CO$_2$ und Licht durchlässige Membran 13 wird genau zentriert über die Oberfläche 14 gespannt unter Zwischenschaltung einer dünnen Schicht des Elektrolyten 8. Der die Membran 13 vorzentriert und gespannt haltende Schnappring 9 ist bekannt und beispielsweise in der DE-A1-30 40 544 beschrieben.

Die lichtemittierenden Dioden 24, 25, bzw. die photoelektrischen Empfängerelemente 32, können an anderer Stelle, beispielsweise im Meßgerät selbst, angeordnet sein und die Lichtzu- bzw. -abführung kann über dünne Glasfasern zur bzw. von der Meßfläche 14 erfolgen.

**Patentansprüche**

1. Kombinationssensor mit einer auf eine Temperatur von T ≤ 42°C thermostatisierten elektrischen Heizeinrichtung (6, 7) zur transkutanen Erfassung von Sauerstoff ($O_2$) und Kohlendioxid ($CO_2$) im Blut und mit einem gemeinsamen Sensorgehäuse (4), in das eine elektrometrische Meßvorrichtung für den $CO_2$-Partialdruck ($pCO_2$) mit einer Iridium/Iridiumoxid-Meßelektrode (11), die durch einen Elektrolyten (8) überdeckt ist, der gegen die Meßstelle auf der Haut durch eine für $CO_2$ und für Licht durchlässige Membran (13) getrennt ist sowie eine Meßvorrichtung (24, 25, 26) zur spektrometrischen Messung der Sauerstoffsättigung des Bluts ($sO_2$) eingebaut sind.

2. Kombinationssensor nach Anspruch 1, **dadurch gekennzeichnet,** daß die Meßvorrichtung zur spektrometrischen Messung zwei in die Meßfläche (14) des Sensors eingesetzte lichtemittierende Dioden (24, 25), die Licht verschiedener Wellenlänge abgeben, und wenigstens einen ebenfalls in die Sensormeßfläche (14) eingesetzten photoelektrischen Empfänger (26) aufweist.

3. Kombinationssensor nach Anspruch 1, **dadurch gekennzeichnet,** daß die Meßvorrichtung zur spektrometrischen $sO_2$-Messung zwei lichtemittierende Dioden aufweist, die Licht verschiedener Wellenlängen und wenigstens ein photoelektrisches Empfängerelement aufweist, und daß die lichtemittierenden Dioden und das Empfängerelement über an der Sensormeßfläche (14) endende Lichtleiter mit dem Sensor verbunden sind.

4. Kombinationssensor nach Anspruch 3, **dadurch gekennzeichnet,** daß die lichtemittierenden Dioden (24, 25) und die photoelektrischen Empfängerelemente (32) mittels einer lichtdurchlässigen Vergußmasse (28, 32) in die Sensormeßfläche (14) eingebettet sind.

5. Kombinationssensor nach Anspruch 3, **dadurch gekennzeichnet,** daß die eine Wellenlänge im Infrarotbereich und die andere Wellenlänge im Rotbereich des elektromagnetischen Spektrums gewählt sind.

6. Kombinationssensor nach Anspruch 5, **dadurch gekennzeichnet,** daß die eine Wellenlänge isosbestisch für Hämoglobin bzw. Oxyhämoglobin und vorzugsweise zu λ = 805 mm und die andere Wellenlänge zu etwa λ = 650 nm gewählt wird.

7. Kombinationssensor nach Anspruch 5 oder 6, **dadurch gekennzeichnet,** daß die lichtemittierenden Dioden (24, 25) vom Überwachungs- und Auswertegerät aus im Takt der Pulsfrequenz intermittierend erregbar sind und daß die Signalauswertung in Anpassung auf die Pulsphasen der arteriellen Füllung des Meßareals erfolgt.

**Claims**

1. Combined sensor, with an electric heating device (6, 7) thermostated at a temperature of T ≤ 42°C, for the transcutaneous measurement of oxygen ($O_2$) and carbon dioxide ($CO_2$) in the blood, and with a common sensor housing (4) in which are incorporated an electrometric measuring device for the partial pressure of $CO_2$ ($pCO_2$) with an iridium/iridium oxide measuring electrode (11) which is covered by an electrolyte (8) which is separated from the measuring site on the skin by a membrane (13) which is permeable to $CO_2$ and light, and a measuring device (24, 25, 26) for the measurement of the blood oxygen saturation ($sO_2$) by spectrometry.

2. Combined sensor according to Claim 1, characterised in that the measuring device for spectrometric measurement has two light-emitting diodes (24, 25) which are inserted in the measuring surface (14) of the sensor and emit light of different wavelengths, and has at least one photoelectric receiver (26) which is likewise inserted in the sensor measuring surface (14).

3. Combined sensor according to Claim 1, characterised in that the measuring device for the spectrometric $sO_2$ measurement has two light-emitting diodes which [lacuna] light of different wavelengths, and has at least one photoelectric receiving unit, and in that the light-emitting diodes and the receiving unit are connected to the sensor by light guides terminating on the sensor measuring surface (14).

4. Combined sensor according to Claim 3, characterised in that the light-emitting diodes (24, 25) and the photoelectric receiving units (32) [sic] are embedded in the sensor measuring surface (14) by a light-transmitting casting composition (28, 32).

5. Combined sensor according to Claim 3, characterised in that one wavelength is chosen to be in the infrared region and the other wavelength is chosen to be in the red region of the electromagnetic spectrum.

6. Combined sensor according to Claim 5, characterised in that one wavelength is chosen to be isosbestic for haemoglobin and oxyhaemoglobin, and is preferably $\lambda = 805$ nm, and the other wavelength is chosen to be about $\lambda = 650$ nm.

7. Combined sensor according to either of Claims 5 or 6, characterised in that the light-emitting diodes (24, 25) can be excited intermittently by the monitoring and evaluating apparatus at a rate equal to the pulse rate, and in that the signal is evaluated in harmony with the pulse phases of the arterial filling of the area measured.

**Revendications**

1. Capteur mixte, comprenant un dispositif de chauffage électrique (6, 7) thermostaté à une température de $T \leqq 42°C$ pour la détection transcutanée d'oxygène ($O_2$) et de dioxyde de carbone ($CO_2$) dans le sang, et un boîtier de capteurs commun (4) dans lequel sont montés un dispositif de mesure électrométrique pour la pression partielle du $CO_2$ ($pCO_2$) et une électrode de mesure iridium/oxyde d'iridium (11) qui est recouverte par un électrolyte (8), lequel est séparé du point de mesure sur la peau par une membrane (13) perméable au $CO_2$ et à la lumière, ainsi qu'un dispositif de mesure (24, 25, 26) pour la mesure spectrométrique de la saturation du sang en oxygène ($sO_2$).

2. Capteur mixte selon la revendication 1, caractérisé en ce que le dispositif pour la mesure spectrométrique comprend deux diodes électroluminescentes (24, 25) insérées dans la surface de mesure (14) du capteur et émettant de la lumière de différentes longueurs d'ondes, et au moins un récepteur photo-électrique (26) également inséré dans la surface de mesure (14) du capteur.

3. Capteur mixte selon la revendication 1, caractérisé en ce que le dispositif de mesure pour la mesure spectrométrique de $sO_2$ comprend deux diodes électroluminescentes qui émettent de la lumière de différentes longueurs d'ondes, et au moins un élément récepteur photo-électrique, et que les diodes électroluminescentes et l'élément récepteur photo-électrique sont reliés au capteur par l'intermédiaire de câbles fibres optiques qui se terminent à la surface de mesure du capteur 14.

4. Capteur mixte selon la revendication 3, caractérisé en ce que les diodes électroluminescentes (24, 25) et les éléments récepteurs photo-électriques (32) sont encastrés dans la surface de mesure (14) du capteur au moyen d'une masse de scellement (28, 32) transparente.

5. Capteur mixte selon la revendication 3, caractérisé en ce que l'une des longueurs d'ondes est choisie dans le domaine infrarouge et l'autre longueur d'onde dans le domaine rouge du spectre électromagnétique.

6. Capteur mixte selon la revendication 5, caractérisé en ce que l'une des longueurs d'ondes pour l'hémoglobine et respectivement l'oxyhémoglobine au point isobestique est choisie de préférence comme étant de $\lambda = 805$ nm, et que l'autre longueur d'onde est choisie comme étant d'environ $\lambda = 650$ nm.

7. Capteur mixte selon l'une des revendications 5 ou 6, caractérisé en ce que les diodes électroluminescentes (24, 25) peuvent être excitées de manière intermittente au rythme du pouls à partir de l'appareil de surveillance et d'analyse, et que l'interprétation des signaux se fait avec adaptation aux phases du pouls du remplissage artériel de la zone de mesure.